# EUROPEAN PATENT SPECIFICATION

(11) **EP 0 403 462 B1**
(45) Date of publication and mention of the grant of the patent: **18.12.1996**
(21) Application number: 90870071.9
(22) Date of filing: 14.05.1990
(51) Int. Cl.: C07C 5/32, C07B 35/04, B01J 23/22

(54) **Process for the catalytic dehydrogenation of hydrocarbons**
Verfahren zur katalytischen Dehydrierung von Kohlenwasserstoffen
Procédé de déshydrogénation catalytique d'hydrocarbures

(30) Priority: 12.05.1989 BE 8900512; 12.05.1989 BE 8900513; 12.05.1989 BE 8900514; 12.05.1989 BE 8900515
(43) Date of publication of application: 19.12.1990
(73) Proprietor: FINA RESEARCH S.A., B-7181 Seneffe (Feluy) (BE)
(72) Inventor: Delorme, Luc Florent Léon, B-1410 Waterloo (BE); Martins Mendes Cerejo, Francisco, B-7190 Ecaussines (BE); Grootjans, Jacques, François, B-3061 Leefdaal (BE)

(56) References cited:
- FR-A- 827 068
- GB-A- 840 082
- US-A- 3 118 007
- US-A- 4 607 129
- US-A- 4 644 089

## Description

The present invention relates to a process for the catalytic dehydrogenation of hydrocarbons. More particularly the process of the invention uses catalytic systems of the redox type. Particularly the present invention relates to the dehydrogenation of paraffinic hydrocarbons to produce the corresponding olefinic hydrocarbons and possibly the corresponding diolefinic hydrocarbons or to the dehydrogenation of olefinic hydrocarbons to produce the corresponding diolefinic hydrocarbons.

Catalytic dehydrogenation of hydrocarbons has been carried out for many years and constitutes an important catalytic process in view of the increasing demand in dehydrogenated products which may be valorized under the most various forms such as high octane gasolines, plastic materials and synthetic rubbers.

In the field of dehydrogenation of alkylaromatic hydrocarbons, known processes include thermal dehydrogenation, catalytic dehydrogenation in the presence of an inert diluent such as steam, and oxidative dehydrogenation, the latter involving the injection of molecular oxygen in the reaction medium. However, although the oxidative dehydrogenation may have same advantages regarding reaction yield and selectivity of the desired product, it is also well known that the presence of molecular oxygen in the reaction medium leads to the formation of indesirable oxidation products such as aldehydes.

In view to partially obviate these drawbacks, it has often been proposed to use very specific catalysts having a particular selectivity towards oxidative dehydrogenation of certain hydrocarbons whether or not of the alkylaromatic type.

In this respect, it has already been proposed in US patent 4,777,319 to Kung et al. to use vanadates or mobybdates of metals for the selective dehydrogenation of paraffinic hydrocarbons having from 3 to 6 carbon atoms. However, the dehydrogenation reaction is necessarily carried out in the presence of molecular oxygen, which displaces the thermodynamic equilibrium but leads to the formation of secondary oxidation products.

It has also been proposed in US 4,742,180 to Gaffney to use supported catalysts, the support being essentially an oxide of praseodymium on which there is deposited an alkaline metal having a dehydrogenating action. However, the availability of praseodymium oxide for the production of huge amounts of dehydrogenated products has to be taken into account. Moreover, it has to be noted that no significant result is indicated for the dehydrogenation of hydrocarbons. Oganowski has also proposed to use a catalyst of the Mg₃(VO₄)₂ type for the dehydrogenation of ethylbenzene, but the reaction necessarily occurs in the presence of a gas containing molecular oxygen. Moreover, it is known that oxides of the V₂O₅ type lead to reaction of complete combustion when used in dehydrogenation reaction of hydrocarbons carried out without bringing in molecular oxygen.

US 4,607,129 discloses a process for the catalytic dehydrocyclization and dehydrogenation of hydrocarbons. The example 5 illustrates the dehydrogenation of propane and isobutane on a calcined and redox-treated V₂O₅/SiO₂ catalyst to primarily propylene and isobutylene respectively.

An object of the present invention is to provide an improved process for the catalytic dehydrogenation of hydrocarbons in the presence of a redox catalytic system.

An object of the present invention is to provide an improved process for the catalytic dehydrogenation of hydrocarbons such as paraffinic hydrocarbons or olefinic hydrocarbons.

Another object of the present invention is to provide an improved process for the catalytic dehydrogenation of hydrocarbons in the absence of molecular oxygen.

A further object of the present invention is to provide an improved process for the catalytic dehydrogenation of hydrocarbons in the presence of a redox catalytic system and of which one or more oxidation stages show a dehydrogenating activity.

Still a further object of the present invention is to provide a continuous process for the dehydrogenation of hydrocarbons in the presence of a redox catalytic system.

The present invention provides for a process for the catalytic dehydrogenation of C₂-C₂₀ paraffinic hydrocarbons, C₃-C₂₀ olefinic hydrocarbons and mixtures thereof into corresponding dehydrogenated hydrocarbons which comprises:
- contacting the hydrocarbon feedstock to be dehydrogenated under dehydrogenation conditions, in the absence of added water vapour and of any gas containing molecular oxygen, with a catalyst consisting of at least one reducible oxide of a metal (I) being vanadium supported on a material selected from clays, zeolitic materials of the metallo-silicate or metallo-alumino-phosphate type, and oxides of a metal (II) selected from Zn, Mg, Ca and Ba, said catalyst having a dehydrogenation activity when said metal (I) has a valency such that it is not in its most reduced state,
- recovering the dehydrogenated hydrocarbons,
- regenerating the used catalyst, and
- contacting the regenerated catalyst with fresh hydrocarbon feedstock to be dehydrogenated.

The Applicant has now found that with the process of the invention, the presence of the molecular oxygen to perform the dehydrogenation reaction was no longer necessary, this fact constituting an important advantage over the prior processes.

According to the process of the invention, the feedstock of hydrocarbons to be dehydrogenated is contacted with a catalyst which has to fulfil several conditions. The catalyst comprises at least a reducible oxide of a metal being vanadium; reducible oxides as used herein mean the hereabove metal oxides which are reduced by contact with hydrocarbons, when operating under dehydrogenation conditions. Moreover, the metal oxide used has to have a dehydrogenating action under the reaction conditions.

Further, the Applicant has found that it is advantageous that these oxides be deposited on a support. By way of examples of suitable supports, it may be cited the oxides of metals selected from Zn, Mg, Ca and Ba as well as clays, and zeolitic materials of the metallo-silicate or metallo-alumino-phosphate type. Within the latter type, it may be cited the alumino-silicates, the borosilicates, silico-alumino-phosphates and other analogs.

The supported catalysts may be prepared according to usual methods such as absorption, precipitation or still impregnation.

Among the various catalysts which may be used in the process of the invention, the Applicant has found that favourable results are obtained with catalysts of the vanadium oxide type deposited on a support comprising magnesium oxide.

The contact between the feedstock to be dehydrogenated and the catalyst may be performed according to different ways; for instance the catalyst particles may be used in a fixed bed, or they may be used in a fluidized bed reactor wherein said particles are circulated and thereafter recovered, what implies a suitable distribution of the particles sizes.

According to an embodiment of the process of the present invention the catalyst is placed in a fixed bed and contacted with the feedstock of hydrocarbons to be dehydrogenated, in the absence of a molecular oxygen containing gas, at a temperature comprised between 360 and 800°C, in order to maintain the feedstock in the vapor phase. When the activity of the catalyst is reduced over the limits which are currently acceptable, generally a reduction of about 10% of the conversion, the feedstock is no longer contacted with the catalyst, but an air stream is passed over the catalyst bed at a temperature of from 200°C to 1000°C in order to regenerate said catalyst under mild conditions. This regeneration comprises at least a mild oxidation of the catalyst. When the catalyst is regenerated the feedstock is again contacted with the catalyst.

According to a preferred embodiment of the present invention, the catalyst particles, whose sizes are comprised between 0.02 and 0.3 mm, the catalyst being taken in its oxidized form, are circulating in the dehydrogenation reaction zone, and contacted with the feedstock to be dehydrogenated, in the absence of an oxygen containing gas, at a temperature comprised between 360 and 800°C in order to maintain the feedstock in the vapor phase. The gas pressure at the outlet of the reactor is generally comprised between 10⁵ and 1.3 10⁵ Pa while residence time of the feedstock in the reactor is of 0.5 to 15 seconds, while the residence time of the catalyst is comprised between 0.5 second and 5 minutes; the upper limit of the residence time of the catalyst depends obviously on its activity. According to this preferred embodiment, the dehydrogenation reaction and the transportation of the catalyst to the regenerator are more particularly carried out in a fluidized bed reactor.

The catalyst in the reactor effluent is separated from the hydrocarbon effluent by suitable means. The separated catalyst is a reduced catalyst because it is in a lower oxidation state than that of the fresh catalyst which enters the reaction zone. The separated catalyst is sent to a regeneration zone where it is regenerated with gaseous stream containing molecular oxygen. This regeneration comprises at least a mild oxidation of the catalyst. The temperature in the regeneration zone is most often maintained between 200 and 1000°C; the residence time of the catalyst in said zone is of 5 seconds to 5 minutes, while that of the gas containing molecular oxygen is of 1 to 30 seconds. The amount of gas together with the oxygen concentration must be sufficient to reoxidize the catalyst in its initial form. If this embodiment is used for the regeneration of the catalyst, then the dehydrogenation process may be carried out continuously, while it was not the case with the previous embodiment.

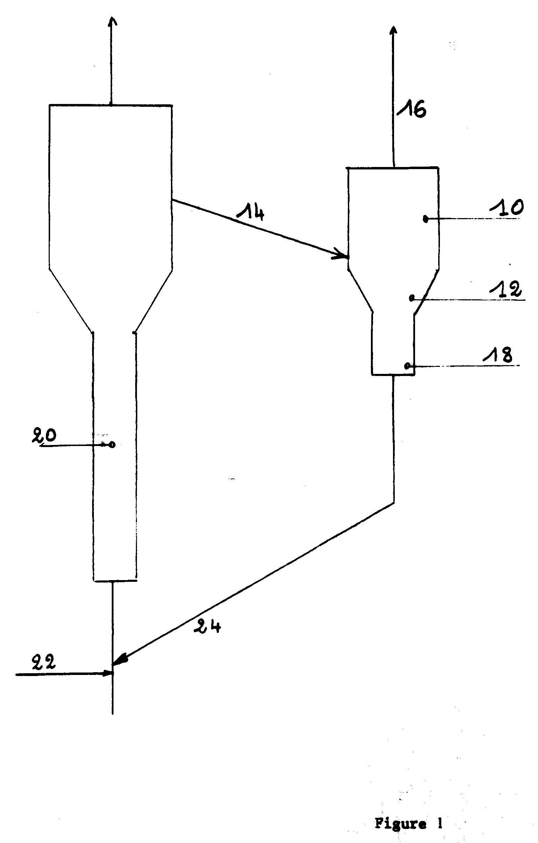

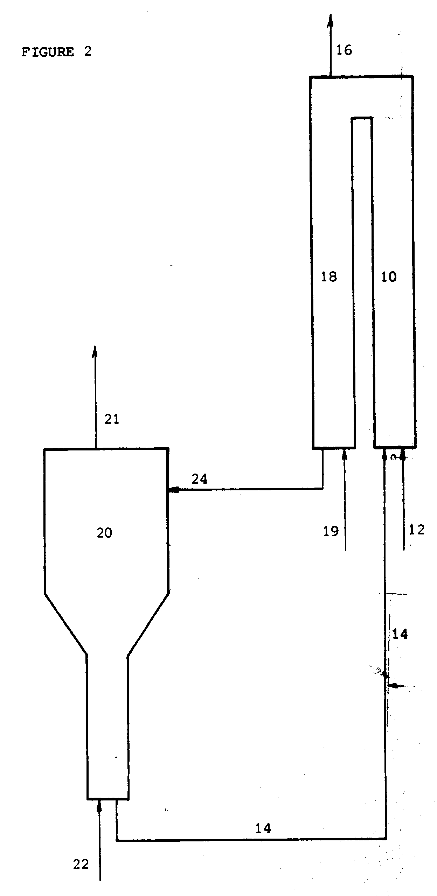

The process of the invention is suitable for the dehydrogenation of C₂-C₂₀ paraffinic hydrocarbons and C₃-C₂₀ olefinic hydrocarbons. Particularly, the process of the invention is applied to the catalytic dehydrogenation of ethane into ethylene, of propane into propylene, of butane into butene, or of butene into butadiene. The process is generally carried out at a temperature comprised between 60 and 800°C and preferably between 400 and 650°C, at a pressure comprised between 0.001 and 1 MPa, and at a hourly space velocity comprised between 0.01 and 20.0 kg of hydrocarbon per hour and per kg of catalyst, preferably between 1 and 10.
Preferred embodiments of the invention are also described by way of the drawings according to which Figures 1 and 2 represent schematic diagrams of the reaction zone and the regeneration zone of the catalyst.

Referring now to Figure 1, which shows the dehydrogenation reaction zone 10 wherein the feedstock of hydrocarbons to be dehydrogenated enters through pipe 12 while the catalyst under its oxidized form enters through pipe 14. The dehydrogenated hydrocarbons are withdrawn through pipe 16 while the reduced catalyst is collected in the area 18 of the dehydrogenation reactor and transported to the top of the regeneration reactor 20. The gas containing molecular oxygen is introduced into pipe 22 while the reduced catalyst is introduced into pipe 24. The catalyst is recovered in the outlet pipe 14 and transported under its oxidized form in the dehydrogenation reactor 10.

Referring to Figure 2, the feedstock of hydrocarbons to be dehydrogenated enters the dehydrogenation zone 10 through pipe 12 while the catalyst under its oxidised form enters through pipe 14. The reduced catalyst is separated in the area 18 of the reactor, using e.g. a nitrogen or steam flow entering through pipe 19. The dehydrogenated feed is withdrawn through pipe 16, while the reduced catalyst is collected into pipe 24 and transported to the top of the regeneration reactor 20. Gas containing molecular oxygen is introduced into reactor 20 through pipe 22 and exits through pipe 23, while the oxidized catalyst is recovered in the outlet pipe 14 and recycled into the dehydrogenation reactor 10.

The process of the present invention shows many advantages with respect to the usual processes and particularly it enables to avoid drastically the formation of oxygenated products, due to the fact that the presence of molecular oxygen is excluded. Moreover, hydrogen is eliminated upon its formation, which enables to displace the equilibrium of the reaction and to run at less high temperatures.
Therefore, it is no more necessary to use diluents such as inert gases to displace the equilibrium; however the process of the invention may also be carried out in the presence of usual diluents.

Another non-negligible advantage of the process of the invention resides in the fact that it allows to work with quasi isothermal reactors at a less high temperature, while with the prior processes it was practically necessary to work under adiabatic conditions.

A substantial increase of the conversion and the yield in dehydrogenated hydrocarbons is also noted. Further, it has been found that it was no more necessary to work under vacuum.

The process of the invention will be better illustrated by way of the following examples.

### Example A

### Preparation of the catalyst

11.21 g of ammonium metavanadate were dissolved in 200 ml of hot deionised water.
30 g of magnesium oxide powder were homogeneized into 200 ml of water at 90°C during 1 hour. The metavanadate solution was then added, and the resulting mixture was homogeneized at 90°C for a further hour.

The mixture temperature was then raised to 120°C and the excess water was evaporated during 18 hours under a nitrogen flow.

The resulting solid catalyst was calcined at 600°C during 4 hours and pelletized. The pellets were ground and sieved to give a V₂O₅-type catalyst supported on magnesium oxide.

### Example 1

Butene-1 was passed over 500 mg catalyst (as obtained from example A) at a temperature of 580°C, under atmospheric pressure, the amount of butene-1 being of 0.95 mg per pulse.
The following results were obtained :
- conversion of butene-1 : 96.8 mol %
- butadiene 1,3 selectivity : 72.3 mol %
- yield : 70.0 %

When the conversion was reduced by about 10% with regard to the initial conversion, butene-1 injections were stopped and the catalyst was regenerated by passing air in pulse mode at a temperature of 500°C.

After regeneration, butene-1 injections were resumed under the same conditions, and the following results were obtained.
- conversion of butene-1 : 95.4 mol %
- butadiene 1,3 selectivity : 72.5 mol %
- yield : 69.1 %

### Example 2

Propane was passed over 500 mg catalyst (as obtained from example A)at a temperature of 580°C, under atmospheric pressure, the amount of propane being of 0.7 mg per pulse.
The following results were obtained.
- conversion of propane : 49.7 mol %
- propylene selectivity : 89.0 mol %
- yield : 42.2 %

When the conversion was reduced by about 10% with regard to the initial conversion, propane injections were stopped and the catalyst was regenerated by passing air in pulse mode at a temperature of 500°C.

After regeneration, propane injections were resumed under the same conditions, and the following results were obained.
- conversion of propane : 48.2 mol %
- propylene selectivity : 89.5 mol %
- yield : 43.1 %

For comparison, a standard Pt-Sn catalyst for dehydrogenation of propane, described as catalyst A in Belgian Patent Application n° 8800157, was used at a temperature of 600°C, under a pressure of 0,11 MPa and with a hourly space velocity by weight of 3.
- propylene selectivity : 96.7%
- yield : 26.0%

This comparative example shows that a higher yield is obtained by the process of the invention, at lower temperatures.

### Examples 3 and 4

The procedure of example 2 was repeated, with catalysts prepared as described in example A except that there was used a solution of 5.99 g ammonium metavanadate in 100 ml of hot deionized water (example 5) or of 1.41 g in 50 ml (example 6).

The following results were obtained :

| | example 5 | example 6 |
|---|---|---|
| - propane conversion (mol %) | 48.2 | 37.2 |
| - propylene selectivity (mol %) | 75.2 | 71.4 |
| - yield (mol %) | 36.2 | 26.6 |

## Claims

1. Process for the catalytic dehydrogenation of C₂-C₂₀ paraffinic hydrocarbons, C₃-C₂₀ olefinic hydrocarbons and mixtures thereof into the corresponding dehydrogenated hydrocarbons, characterized in that it comprises the steps of
- contacting the hydrocarbon feedstock to be dehydrogenated under dehydrogenation conditions, in the absence of added water vapour and of any gas containing molecular oxygen, with a catalyst consisting of at least one reducible oxide of a metal (I) being vanadium, supported on a material selected from clays, zeolitic materials of the metallo-silicate or metallo-alumino-phosphate type, and oxides of a metal (II) selected from Zn, Mg, Ca and Ba, said catalyst having a dehydrogenation activity when said metal (I) has a valency such that it is not in its most reduced state,
- recovering the dehydrogenated hydrocarbons,
- regenerating the used catalyst, and
- contacting the regenerated catalyst with fresh hydrocarbon feedstock to be dehydrogenated.

2. Process according to claim 1, characterized in that the catalyst is supported on magnesium oxide or on zinc oxide.

3. Process according to claim 1, characterized in that the catalyst is supported on a zeolitic material selected from alumino-silicates, borosilicates and silico-alumino-phosphates.

4. Process according to any one of claims 1 to 3, wherein the catalyst recovered from the dehydrogenation reactor is regenerated by sending it into a second reactor wherein a gaseous flow is passed at a temperature of from 200 to 1000°C, with a residence time of from 5 seconds to 5 minutes, and recovered for recycling to the dehydrogenation reactor.

5. Process according to claim 4, wherein the second reactor is of the fluidized bed type and the gaseous flow contains molecular oxygen.

6. Process according to any one of claims 1 to 3, characterized in that the supported catalyst is regenerated by passing on the catalyst bed a gaseous flow containing molecular oxygen at a temperature of from 200 to 1000°C.

7. Process according to 6, wherein the catalyst is regenerated when a reduction of about 10% of the conversion of hydrocarbons is observed.

8. Process according to any one of claims 1 to 7, characterized in that the dehydrogenation reaction is carried out at a temperature of from 360 to 800°C, preferably between 400 and 650°C, at a pressure of from 0.001 to 1 MPa, and with an hourly spatial velocity comprised between 0.01 and 20 kg of hydrocarbon per hour and per kg of catalyst.

## Patentansprüche

1. Verfahren zur katalytischen Dehydrierung von paraffinischen C₂-C₂₀-Kohlenwasserstoffen, olefinischen C₃-C₂₀-Kohlenwasserstoffen und Gemischen davon zu den entsprechenden dehydrierten Kohlenwasserstoffen, dadurch gekennzeichnet, daß es die Schritte umfaßt:
- Inkontaktbringen des zu dehydrierenden Kohlenwasserstoffeinsatzmaterials unter Dehydrierungsbedingungen in Abwesenheit von zugegebenem Wasserdampf und von jeglichem molekularen Sauerstoff enthaltenden Gas mit einem Katalysator, der aus mindestens einem reduzierbaren Oxid eines Metalls (I), nämlich Vanadium, geträgert auf einem aus Tonen, Zeolithmaterialien vom Metallsilicat- oder Metallaluminiumphosphattyp ausgewählten Material, und Oxiden eines Metalls (II), ausgewählt aus Zn, Mg, Ca und Ba besteht, wobei der Katalysator eine Dehydrierungsaktivität aufweist, wenn das Metall (I) eine derartige Valenz aufweist, daß es sich nicht in seinem am stärksten reduzierten Zustand befindet,
- Rückgewinnen der dehydrierten Kohlenwasserstoffe,
- Regenerieren des gebrauchten Katalysators, und
- Inkontaktbringen des regenerierten Katalysators mit frischem, zu dehydrierendem Kohlenwasserstoffeinsatzmaterial.

2. Verfahren nach Anspruch 1, dadurch gekennzeichnet, daß der Katalysator auf Magnesiumoxid oder auf Zinkoxid geträgert ist.

3. Verfahren nach Anspruch 1, dadurch gekennzeichnet, daß der Katalysator auf einem aus Aluminiumsilicaten, Borsilicaten und Siliciumaluminiumphosphaten ausgewählten Zeolithmaterial geträgert ist.

4. Verfahren nach einem der Ansprüche 1 bis 3, wobei der aus dem Dehydrierungsreaktor zurückgewonnene Katalysator durch Einspeisung in einen zweiten Reaktor, durch den ein gasförmiger Strom mit einer Temperatur von 200 bis 1000 °C hindurchgeführt wird, regeneriert wird bei einer Verweilzeit von 5 Sekunden bis 5 Minuten, und zum Recyceln in dem Dehydrierungsreaktor zurückgewonnen wird.

5. Verfahren nach Anspruch 4, wobei der zweite Reaktor vom Typ mit einem Fließbett ist und der gasförmige Strom molekularen Sauerstoff enthält.

6. Verfahren nach einem der Ansprüche 1 bis 3, dadurch gekennzeichnet, daß der geträgert Katalysator dadurch regeneriert wird, daß ein gasförmiger, molekularen Sauerstoff enthaltender Strom mit einer Temperatur von 200 bis 1000 °C über das Katalysatorbett geführt wird.

7. Verfahren nach Anspruch 6, wobei der Katalysator regeneriert wird, wenn eine Verringerung der Umwandlung von Kohlenwasserstoffen von etwa 10 % beobachtet wird.

8. Verfahren nach einem der Ansprüche 1 bis 7, dadurch gekennzeichnet, daß die Dehydrierungsreaktion bei einer Temperatur von 360 bis 800 °C, vorzugsweise zwischen 400 und 650 °C bei einem Druck von 0,001 bis 1 MPa und mit einer Stundenraumgeschwindigkeit zwischen 0,01 und 20 kg Kohlenwasserstoff pro Stunde und pro kg Katalysator durchgeführt wird.

## Revendications

1. Procédé de déshydrogénation catalytique des hydrocarbures paraffiniques en C₂ à C₂₀, des hydrocarbures oléfiniques en C₃ à C₂₀ et de leurs mélanges en hydrocarbures déshydrogénés correspondants qui est caractérisé en ce que:
- l'on met en contact la charge d'hydrocarbures à déshydrogéner dans des conditions de déshydrogénation, en l'absence de vapeur d'eau et de tout gaz contenant de l'oxygène moléculaire, avec un catalyseur constitué par au moins un oxyde réductible d'un métal (I) qui est le vanadium, supporté sur un matériau choisi parmi les argiles, les matériaux zéolitiques du type métallo-silicate ou métallo-alumino-phosphate et les oxydes d'un métal (II) choisi parmi Zn, Mg, Ca et Ba, le dit catalyseur ayant une activité déshydrogénante lorsque le métal (I) est pris à une valence telle qu'il ne soit pas en son état réduit maximum,
- l'on récupère les hydrocarbures déshydrogénés,
- l'on régénère le catalyseur ayant servi, et
- l'on remet le catalyseur régénéré en contact avec la charge fraîche d'hydrocarbures à déshydrogéner.

2. Procédé selon la revendication 1, caractérisé on ce que le catalyseur est supporte sur de l'oxyde de magnésium ou de l'oxyde de zinc.

3. Procédé selon la revendications 1, caractérisé en ce que le catalyseur est supporté sur un matériau zéolitique choisi parmi les alumino silicates, les borosilicates et les silico alumino phosphates.

4. Procédé selon l'une quelconque des revendications 1 à 3, caractérisé en ce que l'on regénère le catalyseur récupéré du réacteur de déshydrogénation en l'envoyant dans un second réacteur dans lequel on le soumet au passage d'un courant gazeux à une température comprise entre 200 et 1000°C, avec un temps de résidence compris entre 5 secondes et 5 minutes, et en récupérant le catalyseur régénéré pour le recycler au réacteur de déshydrogénation.

5. Procédé selon la revendication 4, caractérisé en ce que le second réacteur est à lit fluidisé et que le courant gazeux qui le traverse contient de l'oxygène moléculaire.

6. Procédé selon l'une quelconque des revendications 1 à 3 caractérisé en ce que l'on régénère le catalyseur supporté en faisant passer sur le lit de catalyseur un courant gazeux contenant de l'oxygène moléculaire à une température comprise entre 200 et 1000°C.

7. Procédé selon la revendication 6 caractérisé en ce que l'on effectue la régénération du catalyseur lorsque l'on constate une réduction d'environ 10% de la conversion des hydrocarbures à déshydrogéner.

8. Procédé selon l'une quelconque des revendications 1 à 7, caractérisé en ce que l'on effectue la réaction de déshydrogénation, à une température comprise entre 360 et 800°C, de préférence entre 400 et 650°C, à une pression comprise entre 0,001 et 1 MPa et à une vitesse spatiale horaire comprise entre 0,01 et 20,0 kg d'hydrocarbure par heure et par kg de catalyseur.
